# EUROPEAN PATENT APPLICATION

(11) **EP 2 327 394 A1**
(43) Date of publication of application: **01.06.2011**
(21) Application number: 09382262.5
(22) Date of filing: 26.11.2009
(51) Int. Cl.: A61K 9/08, A61K 31/435

(54) **Liquid pharmaceutical composition**

(71) Applicant: Almirall, S.A., 08022 Barcelona (ES)
(72) Inventor: Duarte Lopez, Begoña, 08980, Sant Feliu de Llobregat (ES); Guiro Coll, Pere, 08980, Barcelona (ES)
(74) Representative: Elzaburu Marquez, Alberto

(57) **Abstract**

Aqueous liquid pharmaceutical composition comprising a) almotriptan and b) at least one compound selected from
i) 1'-methyl-5-((pyrrolidin-1-ylsulfonyl)methyl)spiro[indoline-3,3'-pyrrolidin]-2-ol, and
ii) 3-(2-(dimethylamino)ethyl)-5-((pyrrolidin-1-ylsulfonyl)methyl)-1 H-indol-2-ol.

## Description

### FIELD OF THE INVENTION

The present invention relates to aqueous liquid pharmaceutical compositions comprising almotriptan and particular compounds. Said compositions are stable, thus maintaining the concentrations of almotriptan at constant fixed levels. The invention further relates to a process for the preparation of said compositions and to methods of treatment by administering the compositions.

### BACKGROUND OF THE INVENTION

Migraine is a type of headache, which is a severe, seriously debilitating and usually unilateral form of episodic headache that may be preceded by aura and that is frequently associated with both neurological and gastrointestinal symptoms such as nausea, vomiting, diarrhea, sensitivity to light (photophobia), sound (phonophobia), and smells (osmophobia); sleep disruption, and depression. When untreated, a migraine headache attack may last anywhere from four to 72 hours.

Current migraine treatments are classified as prophylactic treatments or as abortive treatments. Each class of treatments is administered to the migraine sufferer based on the frequency and severity of the headache and its associated symptoms. Prophylactic treatments reduce the frequency of migraines and include non-steroidal antiinflammatory agents, andrenergic beta-blockers, calcium channel blockers, tricyclic antidepressants, selective serotonin reuptake inhibitors, or anticonvulsants.

For occasional migraines, abortive treatments are used. The abortive treatments eliminate or reduce the severity of the headache and any associated symptoms after the migraine has begun. Abortive treatments include serotonin receptor agonists or ergotamine-based compounds, or compounds that provide analgesic effects.

Almotriptan is a member of a family of tryptamine derivatives ("triptans" or "triptan compounds") typically used as abortive medication in the treatment of acute attacks of migraine and cluster headaches. Generally, their action is attributed to their binding to serotonin 5-HT1 B and 5-HT1 D receptors in cranial blood vessels (causing their constriction) and subsequent inhibition of pro-inflammatory neuropeptide release.

Orally administered pharmaceutical compositions are provided to the patient in many dosage forms, including solid forms such as capsules (hard or soft) or tablets (uncoated or coated), and liquid forms such as solutions, emulsions or suspensions. Pharmaceutical compositions administered in solid form are usually intended to be swallowed whole.

Children, the elderly, and many other persons, including disabled or incapacitated patients, often have trouble swallowing tablets or capsules. In these situations, it is desirable to provide the drug either in a chewable solid form or in a liquid form. For many patients, including paediatric and geriatric patients, a liquid oral dosage form is preferable to a chewable dosage form, because of the ease with which it may be swallowed. Additionally, patients may be more inclined to comply with their medication instruction if the dosages are easier to ingest.

However, some active pharmaceutical ingredients (API's) like the triptans, e.g. almotriptan, which are unstable in front of light and/or oxidation, are especially unstable and degrade when present in liquid pharmaceutical compositions which need to be stored at long term and in adverse conditions of temperature and humidity, as recommended in the stability guidelines of the International Conference on Harmonisation of Technical Requirements for Registration of Pharmaceuticals for Human Use (ICH).

Due to this degradation the total amount of almotriptan present in the composition decreases. As a consequence, the patient will receive a lower dose than the one prescribed by the physician.

### SUMMARY OF THE INVENTION

We have now found that liquid pharmaceutical compositions comprising almotriptan and certain particular compounds are stable, ensuring thus that the concentration of almoptriptan is maintained constant at the desired levels.

The aqueous liquid pharmaceutical composition of the invention comprises:
a) almotriptan, or a pharmaceutically acceptable salt or hydrate thereof, and
b) at least one compound selected from
   i) 1'-methyl-5-((pyrrolidin-1-ylsulfonyl)methyl)spiro[indoline-3,3'-pyrrolidin]-2-ol, and
   ii) 3-(2-(dimethylamino)ethyl)-5-((pyrrolidin-1-ylsulfonyl)methyl)-1 H-indol-2-ol.

This composition is stable against light and/or oxidation.

The invention further relates to a composition as defined above for use in the treatment or prevention of migraine and/or cluster headache.

The invention further relates to the use of a composition as defined above for the manufacture of a medicament for the treatment or prevention of migraine and/or cluster headache.

The invention further relates to a method for treating a subject afflicted with migraine and/or cluster headache, which comprises administering to said subject an effective amount of an aqueous liquid pharmaceutical composition as defined above.

Also provided is the use of compound i) 1'-methyl-5-((pyrrolidin-1-ylsulfonyl)methyl)spiro[indoline-3,3'-pyrrolidin]-2-ol, and/or compound ii) 3-(2-(dimethylamino)ethyl)-5-((pyrrolidin-1-ylsulfonyl)methyl)-1 H-indol-2-ol to improve the stability of almotriptan.

### DETAILED DESCRIPTION OF THE INVENTION

### Almotriptan

Almotriptan (3-[2-(Dimethylamino)ethyl]-5-(pyrrolidin-1-ylsulfonylmethyl)-1 H-indole) is a member of a family of tryptamine derivatives ("triptans" or "triptan compounds") typically used as abortive medication in the treatment of migraine and cluster headaches. Generally, their action is attributed to their binding to serotonin 5-HT1 B and 5-HT1 D receptors in cranial blood vessels (causing their constriction) and subsequent inhibition of pro-inflammatory neuropeptide release.

As used herein, the term almotriptan refers to the compound of formula (1) 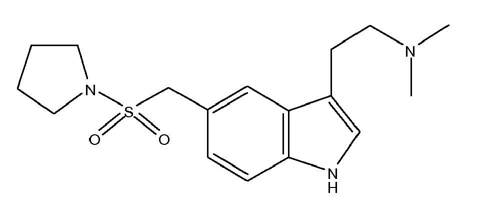 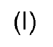 and pharmaceutically acceptable salts or hydrates thereof.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X⁻) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, benzoate, methanesulphonate and *p*-toluenesulphonate.

A preferred pharmaceutically acceptable salt of almotriptan is almotriptan D,L hydrogen malate.

### Compound i)

### 1'-methyl-5-((pyrrolidin-1-ylsulfonyl)methyl)spiro[indoline-3,3'-pyrrolidin]-2-ol corresponds to formula (II)

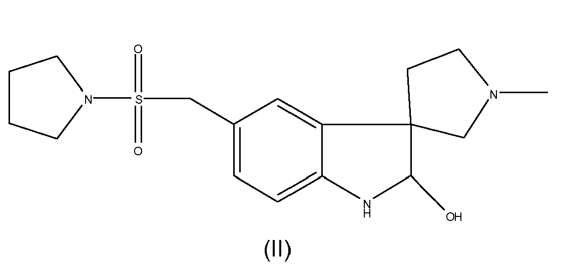

### Compound ii)

### 3-(2-(dimethylamino)ethyl)-5-((pyrrolidin-1-ylsulfonyl)methyl)-1 H-indol-2-ol corresponds to formula (III)

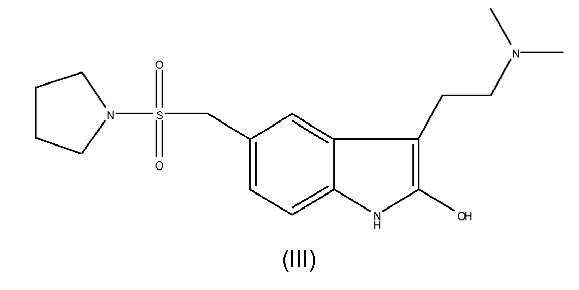

Compound i) and compound ii) can be obtained according to the methods described in Bosch, J.; et al.. Tetrahedron 2001, 57, 1041-1048.

The aqueous liquid pharmaceutical compositions

The present invention provides an aqueous liquid pharmaceutical composition comprising
a) almotriptan, or a pharmaceutically acceptable salt or hydrate thereof, and
b) at least one compound selected from
   i) 1'-methyl-5-((pyrrolidin-1-ylsulfonyl)methyl)spiro[indoline-3,3'-pyrrolidin]-2-ol, and
   ii) 3-(2-(dimethylamino)ethyl)-5-((pyrrolidin-1-ylsulfonyl)methyl)-1 H-indol-2-ol.

Preferred aqueous liquid pharmaceutical compositions of the invention comprise
a) almotriptan, or a pharmaceutically acceptable salt or hydrate thereof, and
b) compound i) 1'-methyl-5-((pyrrolidin-1-ylsulfonyl)methyl)spiro[indoline-3,3'-pyrrolidin]-2-ol, and compound ii) 3-(2-(dimethylamino)ethyl)-5-((pyrrolidin-1-ylsulfonyl)methyl)-1 H-indol-2-ol.

According to the invention, among the suitable pharmaceutically acceptable salts or hydrates of almotriptan, almotriptan D,L hydrogen malate is particularly preferred.

In the aqueous liquid pharmaceutical compositions according to the invention the total amount of a) almotriptan, is preferably in the range of 0.01 to 1 wt.%, more preferably 0.05 to 0.50 wt.%, most preferably 0.06 to 0.30 wt.% based on the total weight of the composition.

The total amount of compound i) 1'-methyl-5-((pyrrolidin-1-ylsulfonyl)methyl)spiro[indoline-3,3'-pyrrolidin]-2-ol is preferably in the range of 0.01 to 1 wt.%, more preferably 0.02 to 0.50 wt.%, most preferably 0.03 to 0.35 wt.%, based on the total weight of almotriptan.

The total amount of compound ii) 3-(2-(dimethylamino)ethyl)-5-((pyrrolidin-1-ylsulfonyl)methyl)-1 H-indol-2-ol is preferably in the range of 0.01 to 1 wt.%, more preferably 0.02 to 0.50 wt.%, most preferably 0.03 to 0.30 wt.%, based on the total weight of almotriptan.

The total amount of compound i) and compound ii) (sum of both compounds) is preferably in the range of 0.01 to 1.5 wt.%, more preferably 0.02 to 1 wt.%, most preferably 0.05 to 0.50 wt.%, based on the total weight of almotriptan.

Preferred aqueous liquid pharmaceutical compositions of the invention comprise,
a) 0.05 to 0.50 wt.%, preferably 0.06 to 0.30 wt.% of almotriptan, pharmaceutically acceptable salts or hydrates thereof, more preferably almotriptan malate, based on the total weight of the composition,
b) 0.02 to 0.50 wt.%, more preferably 0.03 to 0.35 wt.% of compound i) 1'-methyl-5-((pyrrolidin-1-ylsulfonyl)methyl)spiro[indoline-3,3'-pyrrolidin]-2-ol based on the total weight of almotriptan, and / or
   0.02 to 0.50 wt.%, more preferably 0.03 to 0.30 wt.% of compound ii) 3-(2-(dimethylamino)ethyl)-5-((pyrrolidin-1-ylsulfonyl)methyl)-1 H-indol-2-ol, based on the total weight of almotriptan, and
c) balance water up to 100 wt.%.

More preferred aqueous liquid pharmaceutical compositions of the invention comprise,
a) 0.05 to 0.50 wt.%, preferably 0.06 to 0.30 wt.% of almotriptan, pharmaceutically acceptable salts or hydrates thereof, more preferably almotriptan malate, based on the total weight of the composition,
b) 0.02 to 0.50 wt.%, more preferably 0.03 to 0.35 wt.% of compound i) 1'-methyl-5-((pyrrolidin-1-ylsulfonyl)methyl)spiro[indoline-3,3'-pyrrolidin]-2-ol based on the total weight of almotriptan, and
   0.02 to 0.50 wt.%, more preferably 0.03 to 0.30 wt.% of compound ii) 3-(2-(dimethylamino)ethyl)-5-((pyrrolidin-1-ylsulfonyl)methyl)-1 H-indol-2-ol, based on the total weight of almotriptan,
   being the total amount of compound i) and compound ii) (sum of both compounds) in the range of 0.02 to 1 wt.%, preferably 0.05 to 0.50 wt.%, based on the total weight of almotriptan, and
c) balance water up to 100 wt.%.

The amount of almotriptan which is required to achieve a therapeutic effect will, of course, vary with the particular active, the route of administration and the subject under treatment.

To provide a therapeutically effective amount of almotriptan, expressed as its base (not in salt form), it is generally present in an amount of 0.3 mg/mL to 15 mg/mL of the composition, preferably 0.35 mg/mL to 10 mg/mL, and more preferably 0.4 mg/mL to 5 mg/mL. The concentrations expressed herein are based on equivalents of almotriptan base.

The phrase "therapeutically effective amount" means that amount of almotriptan that provides a therapeutic benefit in the treatment or management of migraine and/or cluster headache and general malaise associated therewith.

It is a finding of the present invention that compounds i) and/or ii) are effective in improving the stability of almotriptan. Preferred compositions of the invention are those in which, when stored for one week at 50°C, the almotriptan concentration remains above 90%, preferably above 95%, more preferably above 98%, most preferably above 99% of the initial concentration.

The present invention also provides the use of compounds i) and/or ii) to improve the stability of almotriptan. Preferably, compounds i) and/or ii) are used to reduce the oxidation of almotriptan caused by heat and/or light. Typically, compounds i) and/or ii) are used to improve the stability of almotriptan in an aqueous liquid pharmaceutical composition as defined above.

The aqueous liquid pharmaceutical compositions according to the invention optionally further comprise d) a sweetening agent.

Examples of sweetening agents include sugar sweeteners such as monosaccharides, disaccharides and polysaccharides (xylose, ribose, glucose, mannose, galactose, fructose, dextrose, sucrose, maltose, partially hydrolyzed starch (such as maltitol syrup) or corn syrup solids); sugar alcohols (xylitol, sorbitol, mannitol); other polyols (glycerine, propylene glycol) optionally alkoxylated with ethylene oxide or popylene oxide; artificial sweeteners such as the soluble saccharin salts, i. e., sodium, or calcium saccharin salts, cyclamate salts, acesulfam-K, ammonium glycyrrhizinate, dipotassium glycyrrhizinate and the like, and the free acid form of saccharin; and dipeptide based sweeteners such as L-aspartylphenylalanine methyl ester. Preferred sweeteners are xylitol and mannitol, more preferred xylitol.

The pH value of the aqueous liquid pharmaceutical compositions according to the invention is typically within the acceptable range for physiological administration, and is preferably in the range of 3.0 to 7.0, more preferably in the range of 4 to 6.

The aqueous liquid pharmaceutical compositions according to the invention may optionally further comprise flavouring agents, wetting agents, solubilizing agents, thickening agents, colorant agents, preservative agents, buffering agents or mixtures thereof.

A "flavouring agent" as used herein is a substance capable of enhancing taste or aroma of the aqueous liquid pharmaceutical compositions according to the invention, making then more pleasant. Suitable flavouring agents can be selected from standard reference books, for example Fenaroli's Handbook of Flavor Ingredients, 5th edition (2004).

Flavouring agents may include natural flavouring agents, nature-identical flavouring agents, artificial flavouring agents, or mixtures thereof.

Natural flavouring substances are flavouring substances obtained from plant or animal raw materials, by physical, microbiological or enzymatic processes. They can be either used in their natural state or processed for human consumption. Natural flavouring agents may include, but not limited to, oils derived from plants and fruits such as peppermint oils, spearmint oils, mentha arvensis oils, aniseed oils, clove oils, citrus oils, cinnamon bark oils, strawberry oils, tangerine oils, orange oils, winter green oils, cassia oils, davana oils, spruce needle oils, eucalyptus oils, fennel oils, galbanum oils, ginger oils, camomile oils, cumin oils, rose oils, geranium oils, sage oils, yarrow oils, star anise oils, thyme oils, juniper berry oils, rosemary oils, angelica root oils, and fractions of these oils. Among natural flavouring agents it can also be mentioned anethole, caraway, cardamom seed, cherry juice, cherry syrup, cinnamon, citric acid, cocoa, ginger, glycyrrhiza, honey, orange syrup, peppermint, raspberry juice, spearmint, vanilla and vanillin.

Nature-identical flavouring agents are flavouring substances that are obtained by synthesis or isolated through chemical processes, which are chemically identical to flavouring substances naturally present in products intended for human consumption. Artificial flavouring agents are flavouring substances not identified in a natural product intended for human consumption, whether or not the product is processed.

Flavouring agents can be used singly or in combinations of two or more. Additionally, natural, nature-identical and artificial flavouring agents may be combined in any sensorially acceptable fashion.

Flavouring agents can be used in powder form, in the form of liquid preparations, in the form of suspensions, in the form of elixirs or in the form of syrups containing said flavouring agents.

Flavouring agents can be combined with aroma substances. Examples of homogeneous aroma substances are, for example: anethole, menthol, menthone, isomenthone, menthyl acetate, menthofuran, mint lactone, eucalyptol, limonene, eugenol, pinene, sabinene hydrate, 3-octanol, carvone, gamma-octalactone, gamma-nonalactone, germacrene-D, viridiflorol, 1,3E,5Z-undecatriene, isopulegol, piperitone, 2-butanone, ethyl formate, 3-octyl acetate, isoamyl isovalerate, hexanol, hexanal, cis-3-hexenol, linalool, alpha-terpineol, cis and trans carvyl acetate, p-cymol, damascenon, damascone, rose oxide, dimethyl sulfide, fenchol, acetaldehyde diethyl acetal, cis-4-heptenal, isobutyraldehyde, isovaleraldehyde, cis-jasmone, anisaldehyde, methyl salicylate, myrtenyl acetate, 2-phenylethyl alcohol, 2-phenylethyl isobutyrate, 2-phenylethyl isovalerate, cinnamaldehyde, geraniol, nerol. In the case of chiral compounds, the aroma substances can be present as racemate or an individual enantiomer or as enantiomer-enriched mixture.

Flavouring may include a cooling agent to enhance the flavor and perceived breath freshening of the product. Cooling agents include menthol, ethyl p-menthane carboxamide, N,2,3-trimethyl-2-isopryl-butanamide, menthyl glutarate, menthyl succinate, menthol PG carbonate, menthol EG carbonate, menthyl lactate, menthone glyceryl ketal, menthol glyceryl ether, N-tertbutyl-p-menthane-3-carboxamide, p-menthane-3-carboxylic acid glycerol ester, methyl-2-isopryl-bicyclo, heptane-2-carboxamide, menthol methyl ether and combinations thereof.

Examples of wetting agents (chemical substances that increase the spreading and penetrating properties of a liquid by lowering its surface tension) include one or more cationic surfactants, such as benzalkonium chloride; non-ionic surfactants such as polyoxyethylene and polyoxypropylene block copolymers; polyoxyethylene fatty acid glycerides and oils (such as polyoxyethylene (6) caprylic/capric mono- and diglycerides), polyoxyethylene (40) hydrogenated castor oil; polyoxyethylene sorbitan esters, such as polysorbate 20 and polysorbate 80; propylene glycol fatty acid esters, such as propylene glycol laureate; glyceryl fatty acid esters, such as glyceryl monostearate; sorbitan esters, such as sorbitan monolaurate, sorbitan monooleate, sorbitan monopalmitate and sorbitan monostearate; glyceryl fatty acid esters, for example glyceryl monostearate; or anionic surfactants such as sodium lauryl sulphate, sodium lauryl ether sulphate; fatty acids and salts thereof, such as oleic acid, sodium oleate and triethanolamine oleate.

Examples of solubilizing agents are, for example, nonionic surfactants from at least one of the following groups: products of the addition of 1 to 30 moles of ethylene oxide and/or 0 to 5 moles of propylene oxide onto linear C₈-C₂₂ fatty alcohols, C₁₂-C₂₂ fatty acids and alkyl phenols containing 8 to 15 carbons in the alkyl group; alkyl and/or alkenyl oligoglycosides containing 8 to 22 carbons in the alkyl group and ethoxylated analogs thereof; addition products of 1 to 15 moles of ethylene oxide with castor oil and/or hydrogenated castor oil; addition products of 15 to 60 moles of ethylene oxide with castor oil and/or hydrogenated castor oil; partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide; mixtures of alkoxylated glycerides and alkoxylated glycerine, partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3 to 18 carbons and addition products thereof with 1 to 30 moles of ethylene oxide; mixed esters of pentaerythritol, fatty acids, citric acid and fatty alcohol and/or mixed esters of fatty acids containing 6 to 22 carbons, methyl glucose and polyols, preferably glycerol or polyglycerol; mono-, di- and trialkyl phosphates and mono-, di-and/or tri-PEG-alkyl phosphates and salts thereof; block copolymers, for example Polyethyleneglycol-30 Dipolyhydroxystearate; polymer emulsifiers; polyalkylene glycols and alkyl glyceryl ethers.

A thickening agent or viscosity-enhancing agent can be included to generally thicken the liquid pharmaceutical compositions. While any suitable thickening agent can be included in the compositions of the present invention, a preferred thickening agent, when used, includes one or more of acacia, alginic acid bentonite, carbomer, carboxymethylcellulose calcium or sodium, cetostearyl alcohol, methyl cellulose, ethylcellulose, glycerin, gelatin guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium starch glycolate, starch tragacanth, and xanthan gum, and any combination thereof. More preferred thickening agents are glycerin, hydroxypropylmethylcellulose, and xanthan gum, and any combination thereof.

Optionally, the aqueous liquid pharmaceutical compositions according to the invention further comprise a colouring agent. Suitable colouring agents illustratively include FD & C Red No. 3, FD & C Red No. 20, FD & C Red No. 40, FD & C Yellow No. 6, FD & C Blue No. 2, D & C Green No. 5, D & C Orange No. 5. A full recitation of all FD & C and D & C and their corresponding chemical structures may be found in the Kirk Othmer Encyclopedia of Chemical Technology.

Examples of suitable preservatives to prevent microbial contamination are alkylparabens, particularly methylparaben, propylparaben and butylparaben; sodium benzoate; butylated hydroxy toluene; butylated hydroxyanisole; ethylenediamine tetraacetic acid; chlorobutanol; benzyl alcohol; phenylethylalcohol; dehydroacetic acid; sorbic acid; potassium sorbate; benzalkonium chloride; benzethonium chloride; and mixtures thereof. The amount of preservative generally utilized will vary depending upon the preservative selected.

Any pharmaceutically acceptable buffering agents to adjust the pH of the aqueous liquid pharmaceutical compositions according to the invention to be within the acceptable range for physiological administration, preferably in the range of 3.0 to 7.0, more preferably in the range of 4 to 6, can be used. For example inclusion in the composition of a pharmaceutically acceptable acid such as acetic, citric, fumaric, phosphoric, hydrochloric, lactic or nitric acids or the like, or a mixture thereof. It will also be understood that certain compositions of the invention can have a pH in the desired range without inclusion of a pH adjusting agent specifically for that purpose. Typically, however, an acidic buffer system is present in the composition to achieve the desired pH. An acidic buffer system comprises an acidulant and a buffering agent. Suitable acidulants will be known to those of skill in the art and illustratively include acetic, citric, fumaric, hydrochloric, phosphoric, lactic and nitric acids and the like, and mixtures thereof. Suitable buffering agents will likewise be known to those of skill in the art and illustratively include potassium metaphosphate, potassium phosphate, sodium phosphate, sodium acetate, sodium citrate and the like, and mixtures thereof.

A method for treating a subject afflicted with migraine and/or cluster headache, which comprises administering to said subject an effective amount of an aqueous liquid pharmaceutical compositions according to the invention also forms part of the object of the present invention.

As used herein, the term subject refers to a mammal that may benefit from the administration of a drug composition or method of this invention. Preferably, subject refers to humans.

The invention further relates to the composition of the invention for use in the treatment of the human or animal body, preferably for use in the treatment or prevention of migraine and/or cluster headache. Thus, the invention further relates to use of a composition as defined above for the manufacture of a medicament for the treatment or prevention of migraine and/or cluster headache.

The aqueous liquid pharmaceutical compositions of the present invention may be administered by any suitable route, e.g. orally (as solutions, suspensions and emulsions, etc); rectal, topical, intranasal administration or by parenteral injection (subcutaneous, intradermic, intramuscular, intravenous, etc.) For parenteral injections, aqueous liquid pharmaceutical compositions can be formulated with polyethyelene glycols. However, the preferred route of administration of the aqueous liquid pharmaceutical compositions of the present invention is oral.

The aqueous liquid pharmaceutical compositions of the present invention may be packaged in any suitable container, such as HDPE bottle packs, PET, or amber glass bottle packs, glass tubing, tubular vials, glass ampoules, thermo-form packaging, (foil) pouches, sachets and stick packs.

The pharmaceutical compositions may conveniently be presented in unit dosage form so that the patient may administer a single dose and may be prepared by any of the methods well known in the art of pharmacy. Preferably said unit dosage form ranges from 3 to 25 mL, more preferably from 5 to 20 mL, even more preferred from 6 to 15 mL.

The following examples are given in order to provide a person skilled in the art with a sufficiently clear and complete explanation of the present invention, but should not be considered as limiting of the essential aspects of its subject, as set out in the preceding portions of this description.

### EXAMPLES

### Example 1 - One week stress stability tests

The chemical stability of several aqueous liquid compositions of almotriptan malate in combination with amounts of compound i) and/or compound ii) in the range of 0.01 wt. % to 1 wt.% based on the total weight of almotriptan was evaluated using a stability screening test during one week at 50°C.

To prepare each composition, 17.5 mg of almotriptan D,L hydrogen malate were weighed and dissolved into purified water in a 10 mL tubular amber vial. To said mixture, compound i), and/or compound ii), and optionally a sweetener, were added. After homogenisation, the compositions were stored 1 week at 50°C.

After one week the concentration of almotriptan in all the compositions remained above 90% of the initial concentration.

## Claims

1. An aqueous liquid pharmaceutical composition comprising a) almotriptan, or a pharmaceutically acceptable salt or hydrate thereof, and b) at least one compound selected from
i) 1'-methyl-5-((pyrrolidin-1-ylsulfonyl)methyl)spiro[indoline-3,3'-pyrrolidin]-2-ol, and
ii) 3-(2-(dimethylamino)ethyl)-5-((pyrrolidin-1-ylsulfonyl)methyl)-1 H-indol-2-ol.

2. The composition according to claim 1, wherein the total amount of a) almotriptan is in the range of 0.01 to 1 wt. %, preferably in the range of 0.05 to 0.50 wt. %, based on the total weight of the composition.

3. The composition according to any one of the preceding claims, wherein the total amount of compound i) 1'-methyl-5-((pyrrolidin-1-ylsulfonyl)methyl)spiro[indoline-3,3'-pyrrolidin]-2-ol is in the range of 0.01 to 1 wt.%, preferably in the range of 0.02 to 0.50 wt.%, based on the total weight of almotriptan.

4. The composition according to any one of the preceding claims, wherein the total amount of compound ii) 3-(2-(dimethylamino)ethyl)-5-((pyrrolidin-1-ylsulfonyl)methyl)-1 H-indol-2-ol is in the range of 0.01 to 1 wt.%, preferably in the range of 0.02 to 0.50 wt.%, based on the total weight of almotriptan.

5. The composition according to any one of claims 1 to 4 comprising,
a) 0.05 to 0.5 wt. % of almotriptan, pharmaceutically acceptable salts or hydrates thereof, based on the total weight of the composition,
b) 0.02 to 0.50 wt.% of compound i) 1'-methyl-5-((pyrrolidin-1-ylsulfonyl)methyl)spiro[indoline-3,3'-pyrrolidin]-2-ol based on the total weight of almotriptan, and / or
0.02 to 0.50 wt.% of compound ii) 3-(2-(dimethylamino)ethyl)-5-((pyrrolidin-1-ylsulfonyl)methyl)-1 H-indol-2-0l, based on the total weight of almotriptan, and
c) balance water up to 100 wt. %.

6. The composition according to any one of the preceding claims, comprising compound i) and compound ii).

7. The composition according to claim 6, wherein the total amount of compound i) and compound ii) (sum of both compounds) is in the range of 0.01 to 1.5 wt.%, preferably in the range of 0.02 to 1 wt.%, based on the total weight of almotriptan.

8. The composition according to any one of the preceding claims, further comprising d) a sweeting agent.

9. The composition according to any one of the preceding claims, wherein the composition has a pH value in the range of 3.0 to 7.0.

10. A composition according to any one of claims 1 to 9 for use in the treatment or prevention of migraine and/or cluster headache.

11. Use of a composition according to any one of claims 1 to 9 for the manufacture of a medicament for the treatment or prevention of migraine and/or cluster headache.

12. A method for treating a subject afflicted with migraine and/or cluster headache, which comprises administering to said subject an effective amount of a composition as defined in any one of claims 1 to 9.

13. Use of i) 1'-methyl-5-((pyrrolidin-1-ylsulfonyl)methyl)spiro[indoline-3,3'-pyrrolidin]-2-ol, and/or ii) 3-(2-(dimethylamino)ethyl)-5-((pyrrolidin-1-ylsulfonyl)methyl)-1 H-indol-2-ol to improve the stability of almotriptan.
